# EUROPÄISCHE PATENTSCHRIFT

(11) **EP 0 545 101 B1**
(45) Veröffentlichungstag und Bekanntmachung des Hinweises auf die Patenterteilung: **07.05.1997**
(21) Anmeldenummer: 92119114.4
(22) Anmeldetag: 07.11.1992
(51) Int. Cl.: C07C 53/08, C07C 51/487

(54) **Verfahren zur Reinigung von Abfallessigsäure**
Process for the purification of waste acetic acid
Procédé de purification d'acide acétique de déchets

(30) Priorität: 05.12.1991 DE 4140082
(43) Veröffentlichungstag der Anmeldung: 09.06.1993
(73) Patentinhaber: HOECHST AKTIENGESELLSCHAFT, 65926 Frankfurt am Main (DE)
(72) Erfinder: Seidel, Andreas, Dr., W-5000 Köln 41 (DE); Hauser, Alfred, Dr., W-5042 Erftstadt (DE); Jägers, Erhard, Dr., W-5353 Bornheim 4 (DE); Prinz, Peter, (W) Hürth (DE)

(56) Entgegenhaltungen:
- EP-A- 0 135 085
- DE-A- 1 568 345
- US-A- 3 084 109

## Beschreibung

Die Erfindung betrifft ein Verfahren zur Reinigung von Essigsäure, die als Abfallprodukt von Pharmaka anfällt und als Verunreinigung Stickstoffverbindungen, beispielsweise N-alkylsubstituierte Harnstoffe oder Amide, N,N-substituiertes 1,2-Ethylendiamin, Cyancarbonsäure sowie Halogenverbindungen wie Acetylhalogenide, Halogenwasserstoff oder Mono-, Di- und Trichloressigsäure, enthält.

In der US-A-4,061,546 wird eine oxidative Reinigungsmethode für Essigsäure beschrieben.
In der DE-C-24 23 079 ist ein Verfahren zur Aufarbeitung von essigsäureanhydridhaltiger Abfallessigsäure beschrieben. Die Reinigung dieser Abfallessigsäure erfolgt durch eine Destillation, nachdem vorher der Anhydridanteil durch Zugabe wäßriger Alkali- oder Erdalkaliacetate oder -Hydroxid verseift wurde. Bei dieser Reinigungsmethode wird auch Nitril und Acetylhalogenid verseift und in der Destillationsblase angereichert.

Diese bekannten Reinigungsmethoden versagen jedoch bei den eingangs aufgezählten Stickstoffverbindungen und schwerverseifbaren Halogenverbindungen.

Überraschend wurde nun gefunden, daß diese Stickstoffverbindungen und Halogenverbindungen aus der Abfallessigsäure durch Destillation entfernt werden können, wenn man die Abfallessigsäure in einem ersten Schritt mit einem komplexbildenden Metall oder einer seiner Verbindungen und einer basischen Verbindung versetzt und diese Mischung bei einer Temperatur zwischen 25 und 118 °C über einen Zeitraum von 1 bis 6 Stunden hält, in einem zweiten Schritt einen Vorlauf abdestilliert und in einem dritten Schritt gereinigte Essigsäure von einem schwerflüchtigen Blasenrückstand als Kopfprodukt abdestilliert.

Das erfindungsgemäße Verfahren kann wahlweise noch dadurch ausgestaltet sein, daß man
a) als komplexbildendes Metall Eisen, Cobalt, Nickel oder Kupfer oder deren Verbindungen in Form der Acetate, Oxide oder Hydroxide einsetzt;
b) als basische Verbindung die Hydroxide, Hydrogencarbonate, Carbonate oder Acetate des Natriums, Kaliums, Magnesiums oder Calciums einsetzt;
c) einen 1 bis 100 molaren Überschuß an Metall oder Metallverbindung, bezogen auf die molare Menge an in der Abfallessigsäure enthaltener Stickstoff- und Halogenverbindung, zusetzt;
d) pro 100 Gewichtsteile Abfallessigsäure 1 bis 10 Gewichtsteile basische Verbindung in Form einer 0,1 bis 1 molaren wäßrigen Lösung zusetzt;
e) den ersten Schritt bei einer Temperatur von 100 bis 118 °C in einem Zeitintervall von 1 bis 4 Stunden durchführt;
f) das komplexbildende Metall mit großer Oberfläche in Form von Draht, Granulat oder Pulver einsetzt;
g) den Vorlauf der Destillation als Azeotrop zusammen mit einem zugesetzten Schleppmittel abzieht.

Die Erfindung wird durch das Beispiel näher erläutert:

### Beispiel

5 kg (81,1 mol) Abfallessigsäure aus einer pharmazeutischen Produktion, die als chlorhaltige Verunreinigungen Acetylchlorid, Chlorwasserstoff und Monochloressigsäure sowie als stickstoffhaltige Verunreinigungen N-Methylacetamid, N,N-Dimethylacetamid und Cyanessigsäure enthielt, wurden mit 0,19 mol Cu(CH₃COO)₂ . H₂O und 0,5 l 0,7 molarer Natronlauge 3 Stunden am Rückfluß erhitzt. In einer Kolonne mit 40 Glockenböden wurden in einer Azeotropdestillation, mit Ethylacetat als Schleppmittel, Wasser sowie leichtsiedende Verunreinigungen als Vorlauf abgetrennt. Anschließend wurden 4,5 kg reine Essigsäure als Kopfprodukt der Kolonne entnommen.

In der Tabelle sind die Qualitäten der Abfallessigsäure, der erfindungsgemäß gereinigten Essigsäure und technisch reiner Essigsäure gegenübergestellt.

**Tabelle**

| | technisch reine Essigsäure | Abfallessig säure | gereinigte Essigsäure |
|---|---|---|---|
| Gehalt an Essigsäure (%) | min. 99,5 | 97,3 | 99,9 |
| Wassergehalt (%) | max. 0,1 | 2,2 | 0,02 |
| Verdampfungsrückstand (mg/kg) | < 30 | 2900 | < 5 |
| Erstarrungstemperatur (°C) | > 16,2 | 13,5 | 16,5 |
| Hazen-Farbzahl (APHA) [Farbspezifikation nach ASTM] | max. 10 | 100 | < 5 |
| KMnO₄-Test (min) | > 15 | < 1 | > 180 |
| Ameisensäure (mg/kg) | 500 | < 100 | < 100 |
| Acetaldehyd (mg/kg) | max. 200 | 8 | 0,2 |
| Schwermetalle (mg/kg) | max. 21 | < 2 | < 2 |
| Gesamthalogen (mg/kg) | < 2 | 240 | < 2 |
| Gesamtstickstoff (mg/kg) | < 2 | 430 | < 2 |

Für den KMnO₄-Test verdünnt man 1,0 ml einer 0,1 gewichtsprozentigen (1 g KMnO₄/l H₂O) KMnO₄-Lösung mit 10 ml Wasser und setzt 5 ml Essigsäure zu. Die Zeit bis zur Entfärbung wird gemessen.

## Patentansprüche

1. Verfahren zur Reinigung von Abfallessigsäure, die als Verunreinigungen Stickstoffverbindungen und Halogenverbindungen enthält, dadurch gekennzeichnet, daß man die Abfallessigsäure in einem ersten Schritt mit einem komplexbildenden Metall wie Eisen, Cobalt, Nickel oder Kupfer oder deren Verbindungen und mit den Hydroxiden, Hydrogencarbonaten, Carbonaten oder Acetaten des Natriums, Kaliums, Magnesiums oder Calciums versetzt und diese Mischung bei einer Temperatur zwischen 25 und 118 °C über einen Zeitraum von 1 bis 6 Stunden hält, in einem zweiten Schritt einen Vorlauf abdestilliert und in einem dritten Schritt gereinigte Essigsäure von einem schwerflüchtigen Blasenrückstand als Kopfprodukt abdestilliert.

2. Verfahren nach Anspruch 1, dadurch gekennzeichnet, daß man als Verbindungen des komplexbildenden Metalls die Acetate, Oxide oder Hydroxide einsetzt.

3. Verfahren nach einem der Ansprüche 1 oder 2, dadurch gekennzeichnet, daß man einen 1 bis 100 molaren Überschuß an Metall oder Metallverbindung, bezogen auf die molare Menge an in der der Abfallessigsäure enthaltener Stickstoffverbindung und Halogenverbindung, zusetzt.

4. Verfahren nach einem der Ansprüche 1 bis 3, dadurch gekennzeichnet, daß man pro 100 Gewichtsteile Abfallessigsäure 1 bis 10 Gewichtsteile basische Verbindung in Form einer 0,1 bis 1 molaren wäßrigen Lösung zusetzt.

5. Verfahren nach einem der Ansprüche 1 bis 4, dadurch gekennzeichnet, daß man den ersten Schritt bei einer Temperatur von 100 bis 118°C in einem Zeitintervall von 1 bis 4 Stunden durchführt.

6. Verfahren nach einem der Ansprüche 1 bis 5, dadurch gekennzeichnet, daß man das komplex bildende Metall mit großer Oberfläche in Form von Draht, Granulat oder Pulver einsetzt.

7. Verfahren nach einem der Ansprüche 1 bis 6, dadurch gekennzeichnet, daß man den Vorlauf der Destillation als Azeotrop zusammen mit einem zugesetzten Schleppmittel abzieht.

## Claims

1. A process for the purification of waste acetic acid which contains, as impurities, nitrogen compounds and halogen compounds, which comprises, in a first stage, adding a complex-forming metal such as iron, cobalt, nickel or copper or their compounds and the hydroxides, hydrogen carbonates, carbonates or acetates of sodium, potassium, magnesium or calcium to the waste acetic acid and keeping this mixture at a temperature between 25 and 118°C over a period of time of 1 to 6 hours, in a second stage, distilling off first runnings and in a third stage distilling off, as the overhead product, purified acetic acid from a poorly volatile still residue.

2. The process as claimed in claim 1, wherein the compounds of the complex-forming metal used are acetates, oxides or hydroxides.

3. The process as claimed in either of claims 1 or 2, wherein a 1 to 100 molar excess of metal or metal compound is added, relative to the molar amount of the nitrogen compound and halogen compound contained in the waste acetic acid.

4. The process as claimed in any one of claims 1 to 3, wherein, per 100 parts by weight of waste acetic acid, 1 to 10 parts by weight of basic compound are added in the form of a 0.1 to 1 molar aqueous solution.

5. The process as claimed in any one of claims 1 to 4, wherein the first stage is carried out at a temperature of 100 to 118°C in a period of time of 1 to 4 hours.

6. The process as claimed in any one of claims 1 to 5, wherein the complex-forming metal having a high surface area is used in the form of wire, granules or powder.

7. The process as claimed in any one of claims 1 to 6, wherein the first runnings of the distillation are withdrawn as an azeotrope together with an added entrainer.

## Revendications

1. Procédé pour la purification d'acide acétique usé, qui contient comme impuretés des composés azotés et des composés halogénés, caractérisé en ce qu'on ajoute à l'acide acétique usé dans une première étape un métal complexant comme le fer, le cobalt, le nickel ou le cuivre ou des composés de ceux-ci et les hydroxydes, hydrogénocarbonates, carbonates ou acétates du sodium, potassium, magnésium ou calcium et on maintient ce mélange à une température comprise entre 25 et 118°C sur une durée de 1 à 6 h, on sépare par distillation dans une deuxième étape un écoulement de tête et on sépare par distillation dans une troisième étape de l'acide acétique purifié d'un résidu de la cornue peu volatil en tant que produit de tête.

2. Procédé selon la revendication 1, caractérisé en ce qu'on utilise comme composés du métal complexant les acétates, oxydes ou hydroxydes.

3. Procédé selon l'une quelconque des revendications 1 ou 2, caractérisé en ce qu'on ajoute un excès de 1 à 100 molaires en métal ou en composé du métal, rapporté à la quantité molaire en composé azoté et composé halogéné contenue dans l'acide acétique usé.

4. Procédé selon l'une quelconque des revendications 1 à 3, caractérisé en ce qu'on ajoute pour 100 parties en poids d'acide acétique usé 1 à 10 parties en poids de composé basique dans la forme d'une solution aqueuse 0,1 à 1 molaire.

5. Procédé selon l'une quelconque des revendications 1 à 4, caractérisé en ce qu'on réalise la première étape à une température de 100 à 118°C sur une durée de 1 à 4 h.

6. Procédé selon l'une quelconque des revendications 1 à 5, caractérisé en ce qu'on utilise le métal complexant avec une surface spécifique élevée dans la forme de fil métallique, de granulé ou de poudre.

7. Procédé selon l'une quelconque des revendications 1 à 6, caractérisé en ce qu'on soutire l'écoulement de tête de la distillation comme azéotrope en association avec un produit d'entraînement ajouté.
